# EUROPEAN PATENT SPECIFICATION

(11) **EP 3 390 954 B1**
(45) Date of publication and mention of the grant of the patent: **19.08.2020**
(21) Application number: 16874121.3
(22) Date of filing: 09.12.2016
(51) Int. Cl.: F41B 15/00, F41H 13/00, A61F 5/37

(54) **HUMAN RESTRAINT DEVICE**
PERSONENRÜCKHALTEVORRICHTUNG
DISPOSITIF DE CONTENTION HUMAINE

(30) Priority: 16.12.2015 AU 2015905222
(43) Date of publication of application: 24.10.2018
(73) Proprietor: Anderson, Frank Malcolm, Brisbane, Queensland 4000 (AU); Schutt, Robert Alec, Brisbane, Queensland 4000 (AU)
(72) Inventor: Anderson, Frank Malcolm, Brisbane, Queensland 4000 (AU); Schutt, Robert Alec, Brisbane, Queensland 4000 (AU)
(74) Representative: Margotti, Herwig Franz
(86) International application number: PCT/AU2016/051206
(87) International publication number: WO 2017/100830

(56) References cited:
- WO-A1-2005/008009
- CA-A1- 2 162 221
- CN-U- 203 657 612
- DE-U1- 9 308 186
- JP-A- 2006 244 433
- JP-A- 2010 091 154
- US-A- 5 893 366
- US-B1- 6 336 656
- US-B1- 6 859 939

## Description

### BACKGROUND TO THE INVENTION

There are many situations where it is necessary to restrain individuals. Disorderly, threatening and dangerous behaviour occurs in hospital emergency rooms, police stations, schools, airports and in many other public and private situations. Injuries can take place when an individual resists being restrained. Those seeking to restrain a person can also be injured during a restraining action.

Restraining individuals typically requires physical contact between the person to be restrained and those seeking to carry out the restraining action. Various non-contact means of restraint are available, including the use of lachrymatic sprays (such as pepper spray) and the like or stronger disabling means such as guns or tasers. However, use of many of these restraining methods involves significant risks of injury both to participants in the restraining action and to bystanders.

Spitting presents significant health risks to hospital staff, police and others when they attempt to restrain persons affected with transmissible diseases. Stand-alone anti-spitting masks are effective but can be difficult to position properly on a person until a person has been restrained.

Persons restrained by handcuffs can attempt to move away from those seeking to restrain them. Convenient and robust means of resisting attempts by restrained persons to move away from his/her restrainers will be advantageous in many situations.

US patent 6,859,939 describes an inflatable restraint for selectively restraining the movement of a person. The inflatable restraint includes an inflation unit, a harness, and a plurality of selectively inflatable chambers connected to the harness. The chambers are strategically positioned around selective parts of a body of the person and held in position by the harness.

When the inflation unit is activated the plurality of selectively inflatable chambers are caused to inflate thereby restricting movement of the selective body parts of the person. However, such a harness needs to be outfitted on the person, in preparation for later restraint.

There is therefore a need for an improved human restraint device.

### SUMMARY OF THE INVENTION

According to one aspect, the invention is a human restraint device, comprising:
a band dimensioned to fit over an upper torso and shoulders of a person;
an inflatable tube attached to an inside wall of the band, wherein the tube expands during inflation inwards and away from the band, and whereby the tube when inflated presses against the torso of the person;
a net comprising a mouth attached around a circumference of the band; and
a gas release mechanism to rapidly inflate the inflatable tube.

Preferably, an anti-spitting mask attached to a pouch end of the net.

Preferably, the device further comprises a rope or plurality of ropes attached to the band or net, wherein in use the rope can be grasped and held by a person or persons other than the person on whom said device is fitted.

Preferably, the mechanism to rapidly inflate the inflatable tube is connected to a cartridge of compressed gas.

Preferably, the compressed gas is carbon dioxide.

Preferably, the gas release mechanism is activated automatically.

Preferably, the inflatable tube is inflated by release of compressed gas from two or more disposable cartridges of compressed gas.

Preferably, the inflatable tube is inflated by gas produced by a gas generator.

Preferably, a plurality of lights is positioned around an outside circumference of the band.

Preferably, a plurality of non-overlapping inflatable tubes is positioned around an inside circumference of the band.

Preferably, the inflatable tube is held in position relative to an inside wall of the band and around the circumference of the band by hook and loop fasteners.

Preferably, the device further comprises a mechanism for deflating the inflatable tube.

Preferably, the device further comprises a rope or a plurality of ropes located around an outside circumference of the band.

Preferably, the net retains the band with the inflatable tube attached at about waist height on the person when the band is passed over the head, arms and torso of the person.

Preferably, the net retains the band with the inflatable tube attached between about hip height to about a height of the rib cage on the person when the band is passed over the head, arms and torso of the person.

Preferably, a depth of the net is between 0.5 metre and 1.0 metre.

Preferably, the band dimensioned to fit over an upper torso and shoulders of a person is dimensioned to have an inner diameter between 0.5 metre and 1.0 metre.

### BRIEF DESCRIPTION OF THE DRAWINGS

To assist in understanding the invention and to enable a person skilled in the art to put the invention into practical effect, preferred embodiments of the invention are described below by way of example only with reference to the accompanying figures.

The figures provided herewith illustrate an arrangement of the components of the device consistent with the text herein that describes the invention. Other arrangements of these components consistent with the description of the device can be depicted. The dimensions in the figures are indicative only and not drawn to scale. The numbers on the figures indicating aspects of the device are used below in the description of these figures. Each feature of the device depicted in the figures uses the same reference number in all figures.
Fig. 1 is a side view of the device, according to one embodiment of the present invention, shown in position on a person.
Fig. 2 is a cross-sectional plan view of the device of Fig. 1, where the cross-section shown is through a band of the device that is in position at about waist height on a person.

### DETAILED DESCRIPTION OF THE PREFERRED EMBODIMENT

The present invention relates to a human restraint device. Elements of the invention are illustrated in concise outline form in the drawings, showing only those specific details that are necessary to understanding the embodiments of the present invention, but so as not to clutter the disclosure with excessive detail that will be obvious to those of ordinary skill in the art in light of the present description.

In this patent specification, adjectives such as first and second, left and right, top and bottom, upper and lower, rear, front and side, etc., are used solely to define one element or method step from another element or method step without necessarily requiring a specific relative position or sequence that is described by the adjectives. Words such as "comprises" or "includes" are not used to define an exclusive set of elements or method steps. Rather, such words merely define a minimum set of elements or method steps included in a particular embodiment of the present invention.

In a preferred embodiment, the invention described herein is a human restraint device to assist restraint of a person by combining in one device means of restricting that person's arm movements, means of stopping that person spitting at those seeking to restrain him/her, and means of stopping attempts by that person restrained by the device from moving away from his/her restrainers.

In the following description of the device, it is to be understood that the arms and torso of a person on whom the device is fitted may be clothed or unclothed. Where the description of the device includes phrases such as 'in contact with a sufficient surface area of the torso' it is to be understood that these parts of the body may be clothed or unclothed.

The present invention exploits the fact that a person can be more easily restrained when his/her arms are held firmly against the torso than when that person is able to fend off attempts of such control by defensive or aggressive use of his/her arms. A person can be more easily restrained when his/her arms, head and torso are inside a net than when that person is able to use his/her arms defensively without being restricted by a net. A person being restrained presents a reduced health risk to those seeking to restrain that person if that person is unable to spit at his/her restrainers.

According to some embodiments, the invention comprises components including: a band dimensioned to fit over the head, shoulders and upper torso of a person; an inflatable tube fitted to the inside wall of the band and around the circumference of the band; a net attached to the circumference of the band; an anti-spitting mask attached to the net; components to inflate and deflate the inflatable tube; and a component that can be grasped and held to stop a person restrained by the device from moving away from his/her restrainers.

The inflatable tube of the device is tied, bonded, or otherwise held in position against to the inside wall of the band of the device around the circumference of that band. The tube is held in this position relative to the band when deflated or inflated. The tube is made of airtight material such as rubber or neoprene. The tube can be made of other airtight materials. The tube can be made of elastic or non-elastic material.

The device is placed on a person when the inflatable tube of the device is deflated. It is placed on a person by passing it over the head, shoulders, arms and upper torso of the person to be restrained and downwards over the torso and arms of that person until the pouch end of the net of the device is firmly positioned on the head of that person and the band of the device is at about waist height on that person.

The device is defined as being in position on a person to be restrained when the band of the device is approximately at waist height on the torso of that person. The inflatable tube is inflated when the device is in position on a person to be restrained, as defined above. When it is inflated, the tube expands inwards from the inside wall of the band of the device towards the torso of the person on whom the device is fitted. The device is removed from a person on whom it is fitted by deflating the inflatable tube and then lifting the device upwards over the torso, arms, shoulders and head of that person.

When the device is in position on a person, as defined above, and the inflatable tube of the device is inflated, the result can be that both arms are held between the inflated tube and that person's torso, or the result can be that one arm is or both arms are within the net of the device and not between the inflated tube and that person's torso. In this way, inflation of the tube directly restricts movement of the arm or arms held between the tube and the torso of the person on whom the device is fitted. The net of the device complements the restraining effect of the inflated tube by restricting movement of a person's arm or arms that are not held between the inflated tube and that person's torso to movement within the net.

The band of a preferred embodiment of the device has cross-sectional dimensions of thickness and width appropriate for fitting to it of the inflatable tube, the net, the components for inflation and deflation of the inflatable tube, and the component that can be grasped and held to stop the person restrained by the device from moving away from his/her restrainers.

In a preferred embodiment of the device, the device can be passed over the head, shoulders, arms and torso of the person to be restrained without the need to reshape the band before it is fitted to the person to be restrained.

The band is made of material that does not deform or break during use of the device. The band is made of material and is shaped so the device presents a low risk of causing injury to the user of the device or injury to the person on whom the device is fitted. High-density polyethylene (HDPE) can be used as the material for the band of the device. The band can be made of other materials.

The length of the circumference of the band is such that when the inflatable tube is inflated that a sufficient area of the surface of the tube is in contact with a sufficient surface area of the torso of the person on whom the device is fitted and of the arm or arms of that person that is/are held between the tube and that person's torso to impose force on those body parts with which it is in contact that is sufficient to restrict movement of that person's arm or arms that is/are between the tube and that person's torso. The force so exerted by the inflated tube restricts upward movement of the device relative to the torso of that person on whom the device is fitted.

In one embodiment of the device, the length of the circumference of the band is variable.

In a preferred embodiment of the device and when the device is in position on a person, as defined above, the inflatable tube is inflated by releasing compressed gas into the tube from a compressed gas cartridge connected to the gas release mechanism of the tube inflation component of the device. The gas release mechanism is attached to the band of the device. The gas release mechanism is activated to release gas from the compressed gas cartridge by manually activating the gas release mechanism. In an embodiment of the device, it is fitted with a means to inflate the inflatable tube automatically when the device is in position on a person, as defined above.

The cross-sectional shape, dimensions and material of the inflatable tube of the device are such that when the tube is inflated it does not injure the person on whom the device is fitted.

The circumference of the mouth of the net of the device, that is the open end of the net, is securely attached to the band of the device around the circumference of that band. In a preferred embodiment of the device, the mouth of the net is attached to the upper part of the circumference of the band of the device so the inflatable tube can be inflated without interfering with the operation of the net or other components of the device.

The net has dimensions and shape such that the device with all components fitted to it can be passed over the head, shoulders, arms and upper torso of the person to be restrained and downwards over the torso until the device is in position on the person, as defined above. The depth of the net is such that the net becomes taut when the band of the device is in position on a person, as defined above. The mesh size of the net is such that the person on whom the device is fitted cannot pass his or her hands through the mesh of the net. The net is made of material of strength sufficient to withstand damage during use.

In a preferred embodiment of the device, the pouch part of the net of the device, that is the end section of the net opposite to the mouth of the net that is attached to the band, has a sufficient area of spit-proof material attached to it to form an anti-spitting mask to stop the person fitted with the device spitting on those restraining him/her while allowing that person to see his/her surroundings and allowing that person to breathe adequately.

The device has a component used to release the gas from the inflatable tube when that tube is to be deflated. In an embodiment of the device, the device can include means of regulating the maximum pressure in the inflatable tube.

In a preferred embodiment of the device, the components of the device to inflate and deflate the inflatable tube are attached to the outside of the band of the device so they do not interfere with the use of the device to restrain a person. In this embodiment of the device, the components to inflate and deflate the inflatable tube are connected to this tube by separate pipes through the wall of the band. The components to inflate and deflate the inflatable tube can be connected to this tube in other ways.

In a preferred embodiment of the device, the compressed gas for inflation of the inflatable tube of the device is stored in a single-use cartridge connectable to the gas release mechanism that releases the compressed gas into the inflatable tube. Carbon dioxide is a suitable compressed gas for use with the device. Cartridges of a different compressed gas that are consistent with safe operation of the device can be used with the device. A compressed gas cartridge from which the gas has been released is removed from the gas release mechanism and replaced by a cartridge that is filled with compressed gas.

Gas in the compressed gas cartridge is released to inflate the tube when the device is in position on the person to be restrained, as defined above. In a preferred embodiment of the device, compressed gas is released from the compressed gas cartridge by pulling an actuation cord fitted to the gas release mechanism of the device. Pulling the actuation cord punctures the gas seal of the compressed gas cartridge and releases the compressed gas from the cartridge to pass through the gas release mechanism into a pipe communicating through the wall of the band of the device with the inflatable tube. Other means of releasing gas into the inflatable tube can be used.

The quantity of gas contained in the compressed gas cartridge used to inflate the inflatable tube is that quantity of gas which when released into the inflatable tube inflates that tube such that the tube imposes the force on the torso of the person on whom the device is fitted and imposes the force on that person's arm or arms that is/are between this tube when inflated and that person's torso that is necessary to restrict movement of the arm or arms of that person so positioned and that holds the device in position on that person, as defined earlier.

The quantity of gas contained in the compressed gas cartridge that is released into the inflatable tube of the device to inflate this tube is less than that quantity of gas that would inflate the inflatable tube to a pressure that could cause injury to the person on whom the device is fitted. An embodiment of the device can be fitted with a pressure regulator to limit the pressure in the inflatable tube of the device.

In a preferred embodiment of the device, the component of the device used to deflate the inflatable tube when it is inflated is fitted to the outside wall of the band of the device and is connected by a pipe through the wall of the band to the inflatable tube. Other configurations of this component can be used to deflate the inflatable tube of the device.

In a preferred embodiment of the device, the mask is attached to the net such that the mask is removable from the net and a replacement mask can be fitted. In the preferred embodiment of the device, the anti-spitting mask component is disposable and replaced after use of the device. In a preferred embodiment of the device the mask is attached to the inside of the net. The anti-spitting mask covering the pouch end of the net is sufficiently flexible and attached to the net such that the pouch end of the net can be pulled down onto the head of the person to be restrained until it is in close contact with the head of that person.

Depending on the material used for the anti-spitting mask, the mask may impair the vision of the person on whom the device is fitted. In a preferred embodiment of the device, the anti-spitting mask allows the person on whom the device is fitted to see adequately his/her surroundings. The material used for the anti-spitting mask allows the person on whom the device is fitted to breathe adequately when the anti-spitting mask is in contact with that person's face parts.

The component of the device used to stop a person restrained by the device from moving away from his/her restrainers comprises a rope or a plurality of ropes located around the outside circumference of the band of the device, and that are connected to the band such that they remain connected to the band if grasped and pulled during use of the device, and such that they do not obstruct the placement of the device on a person to be restrained by the device.

In a preferred embodiment of this component of the device, the rope or a plurality of ropes is positioned and connected to the band of the device around the circumference of this band such that as much as is practicable of the circumference of the band has a length of rope on the outside wall of this band that can be grasped and held by the restrainers seeking to limit movement away from them by the person being restrained by the device. This component of the device can be configured in other ways that are consistent with allowing the restrainers of the person on whom the device is fitted to grasp and hold the device to stop that person from moving away from his/her restrainers.

In a preferred embodiment of the device, the components of the device are shaped and positioned on the device or fitted with protective covering to minimize the risk of injury to either the persons applying the device or the person to be restrained by the device.

The device can be constructed with dimensions of components made according to the size characteristics of the human populations where it is to be used.

In an embodiment of the device, the band of the device is flexible such that the circumference of the band with the inflatable tube attached can require reshaping to allow it to be passed over the head, shoulders, arms and torso of the person to be restrained. In an embodiment of the device, the band of the device to which the inflatable tube is attached is rigid and the band retains its shape when fitted to a person and the inflatable tube is inflated.

In an embodiment of the device, the inflatable tube is held in position relative to the inside wall of the band and around the circumference of the band by a length or lengths of hook and loop fasteners, such as Velcro® brand fasteners. In an embodiment of the device, the inflatable tube is held in position relative to the inside surface of the band by a plurality of straps positioned around the circumference of the band and where each strap binds the inflatable tube to the band at that part of the circumference of the band where the strap is located.

In an embodiment of the device a plurality of non-overlapping inflatable tubes is located around the inside circumference of the band of the device and these inflatable tubes are connected to the gas release mechanism of the device so they inflate at the same time when the compressed gas is released from the compressed gas cartridge into them. In this embodiment of the device, each of the separate inflatable tubes inflates inwards from the inside surface of the band to impose force around the torso of the person on whom the device is fitted and on the arm or arms that are between these tubes and that person's torso. In this embodiment of the device with a plurality of inflatable tubes, all the tubes can be deflated to allow the device to be removed from the wearer of the device.

An embodiment of the device can be fitted with two or more single-use cartridges of compressed gas and gas release mechanisms that together contain the quantity of compressed gas necessary for inflation of the inflatable tube of the device.

In an embodiment of the device, a signal from a suitable radio transmitter is paired with a radio signal receiver on the device to activate the gas release mechanism on the device. Different embodiments of the device can be fitted with different combinations of manually, automatically or remotely activated gas release controls.

In an embodiment of the device, the inflatable tube of the device is inflated by gas produced by a gas generator of the general type used to deploy airbags in vehicles. When such a gas generator is used with the device, the gas generator is charged only with the quantity of gas generating chemicals that is needed to generate the volume of gas required to inflate the inflatable tube to the pressure necessary to make the device safe and effective.

In an embodiment of the device the material of the anti-spitting mask is made such that it forms an opaque barrier. In this embodiment of the device the opaque mask allows the person wearing the device to breathe adequately.

An embodiment of the device has a drawstring fitted to the net around the circumference of the net above the band of the device and approximately at chest height when the device is in position on a person, as defined. An embodiment of the device is fitted with lights around the circumference of the band of the device that are illuminated manually or automatically.

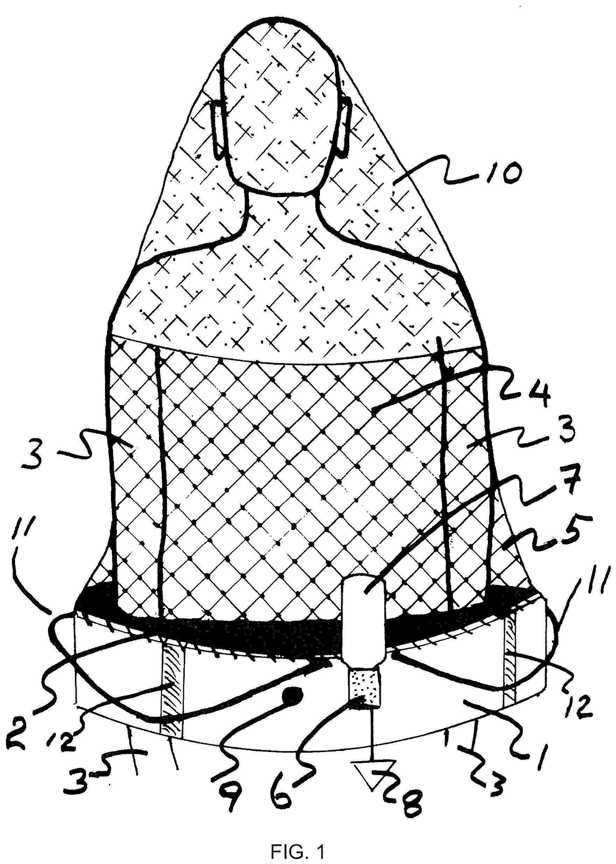

Fig. 1 is a side view of the device, according to one embodiment of the present invention, shown in position on a person. The view is from the back of that person. The band **1** is shown to be at about waist height on the wearer of the device. The inflatable tube **2** shown as inflated and both arms **3** are held between the person's torso **4** and the inflated tube **2.** The net **5** is shown as attached to the upper edge of the band **1** by a rope looping through a plurality of holes in the upper edge of the band **1** and the mesh at the mouth end of the net **5.** The inflatable tube **2** is shown as being between the inside wall of the band **1** and the person's torso **4** and arms **3.**

A gas release mechanism **6** releases compressed gas from the single-use gas cartridge **7** and inflates the inflatable tube **2.** The gas release mechanism **6** of the device is shown as attached to the outside wall of the band **1.** The compressed gas cartridge **7** is shown as fitted into the gas release mechanism **6.** The gas released from the cartridge **7** passes from the gas release mechanism **6** through a pipe (not shown) in the wall of the band 1 connecting the gas release mechanism **6** to the inflatable tube **2.** The gas release mechanism **6** is activated by pulling the handle **8** of the activation cord on the gas release mechanism **6.**

Gas is released from the inflatable tube **2** by activation of the deflation mechanism **9** shown fitted to the outside wall of the band **1.** The deflation mechanism **9** is connected by a pipe (not shown) through the wall of the band **1** to the inflatable tube 2.

The net **5** is shown as covered at a pouch end of the net **5** down to the upper part of the torso **4** by a spit-proof material **10,** such that the person wearing the device is unable to spit on those seeking to restrain him/her.

Ropes **11** that can be grasped to take hold of the device are shown attached to the band **1.** Two straps **12** binding the inflatable tube **2** to the band **1** are shown; additional straps are used on that part of the band **1** not shown in this figure.

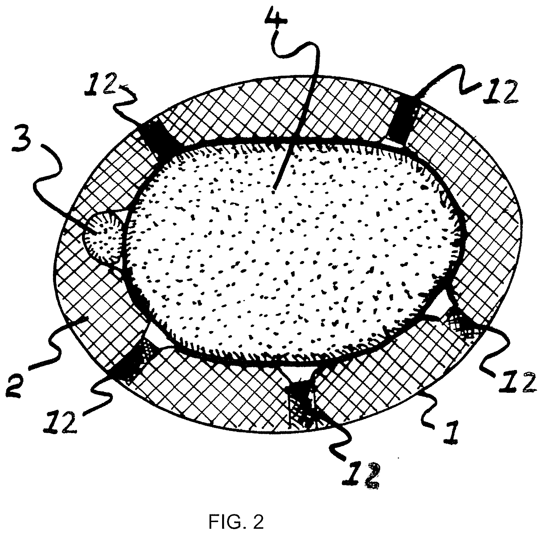

Fig. 2 is a cross-sectional plan view of the device of Fig. 1, where the cross-section shown is through the band **1** of the device that is in position at about waist height on a person. The inflatable tube **2** is shown as inflated and being in contact with the torso **4** and one arm **3** of the person on whom the device is fitted. The second arm will be under the net but not between the inflated tube **2** and the torso **4** of the person on whom the device is fitted. In this embodiment of the device, five straps **12** are shown to hold the inflatable tube **2** in position against the inside wall of the band **1.**

The unshaded areas of Fig. 2 between the inflated tube **2** and arm **3** and the torso **4** are where the straps **12** do not allow the tube **2** to be in contact with the torso **4.** In this figure, the band **1** is depicted as being approximately elliptical and thus conforming approximately to the combined overall shape of the torso **4** plus one arm **3.**

The above description of various embodiments of the present invention is provided for purposes of description to one of ordinary skill in the related art. It is not intended to be exhaustive or to limit the invention to a single disclosed embodiment. Numerous alternatives and variations to the present invention will be apparent to those skilled in the art of the above teaching. Accordingly, while some alternative embodiments have been discussed specifically, other embodiments will be apparent or relatively easily developed by those of ordinary skill in the art. Accordingly, this patent specification is intended to embrace all alternatives, modifications and variations of the present invention that have been discussed herein, and other embodiments that fall within the scope of the above described invention as defined in the appended claims.

## Claims

1. A human restraint device, comprising:
a band (1) dimensioned to fit over an upper torso and shoulders of a person;
an inflatable tube (2) attached to an inside wall of the band (1), wherein the tube expands during inflation inwards and away from the band (1), and whereby the tube (2) when inflated presses against the torso of the person;
a net (5) comprising a mouth attached around a circumference of the band (1); and
a gas release mechanism (6) to rapidly inflate the inflatable tube (2).

2. The restraint device of claim 1, further comprising an anti-spitting mask (10) attached to a pouch end of the net (5).

3. The restraint device of claim 1 or claim 2, further comprising a rope (11) or plurality of ropes attached to the band (1) or net (5), wherein in use the rope (11) can be grasped and held by a person or persons other than the person on whom said device is fitted.

4. The restraint device of any of claims 1 to 3, wherein the mechanism (6) to rapidly inflate the inflatable tube (2) is connected to a cartridge (7) of compressed gas.

5. The restraint device of claim 4, wherein the compressed gas is carbon dioxide.

6. The restraint device of any one of the above claims, wherein the gas release mechanism (6) is activated automatically.

7. The restraint device of any one of the above claims, wherein the inflatable tube (2) is inflated by release of compressed gas from two or more disposable cartridges of compressed gas.

8. The restraint device of any one of the above claims, wherein the inflatable tube (2) is inflated by gas produced by a gas generator.

9. The restraint device of any one of the above claims, further comprising a plurality of lights positioned around an outside circumference of the band (1).

10. The restraint device of any one of the above claims, further comprising a plurality of non-overlapping inflatable tubes positioned around an inside circumference of the band (1).

11. The restraint device of any one of the above claims, wherein the inflatable tube (2) is held in position relative to the inside wall of the band (1) and around the circumference of the band by hook and loop fasteners.

12. The restraint device of any one of the above claims, further comprising a mechanism (9) for deflating the inflatable tube (2).

13. The restraint device of any one of the above claims, further comprising a rope (11) or a plurality of ropes located around an outside circumference of the band (1).

14. The restraint device of any one of the above claims, wherein the net (5) retains the band (1) with the inflatable tube (2) attached at about waist height on the person when the band (1) is passed over the head, arms and torso of the person.

15. The restraint device of any one of claims 1-13, wherein the net retains the band (1) with the inflatable tube (2) attached between about hip height to about a height of the rib cage on the person when the band (1) is passed over the head, arms and torso of the person.

16. The restraint device of any one of claims 1-13, wherein a depth of the net (5) is between 0.5 metre and 1.0 metre.

17. The restraint device of any one of the above claims, wherein the band dimensioned to fit over an upper torso and shoulders of a person is dimensioned to have an inner diameter between 0.5 metre and 1.0 metre.

## Patentansprüche

1. Rückhaltevorrichtung für Menschen, umfassend:
ein Band (1), das so bemessen ist, dass es über den Oberkörper und die Schultern einer Person passt;
einen aufblasbaren Schlauch (2), der an einer Innenwand des Bandes (1) befestigt ist, wobei sich der Schlauch während des Aufblasens nach innen und vom Band (1) weg ausdehnt und wodurch der Schlauch (2) im aufgeblasenen Zustand gegen den Rumpf der Person drückt;
ein Netz (5) mit einer Mündung, die um einen Umfang des Bandes (1) herum befestigt ist; und
einen Gasfreisetzungsmechanismus (6) zum schnellen Aufblasen des aufblasbaren Schlauchs (2).

2. Rückhaltevorrichtung nach Anspruch 1, ferner umfassend eine Spuckschutzmaske (10), die an einem Beutelende des Netzes (5) befestigt ist.

3. Rückhaltevorrichtung nach Anspruch 1 oder Anspruch 2, ferner umfassend ein Seil (11) oder eine Mehrzahl von Seilen, die an dem Band (1) oder Netz (5) befestigt sind, wobei das Seil (11) im Gebrauch von einer anderen Person oder von anderen Personen als jener Person, an der die Vorrichtung angebracht ist, ergriffen und gehalten werden kann.

4. Rückhaltevorrichtung nach einem der Ansprüche 1 bis 3, wobei der Mechanismus (6) zum schnellen Aufblasen des aufblasbaren Schlauchs (2) mit einer Druckgaspatrone (7) verbunden ist.

5. Rückhaltevorrichtung nach Anspruch 4, wobei es sich bei dem Druckgas um Kohlendioxid handelt.

6. Rückhaltevorrichtung nach einem der obengenannten Ansprüche, wobei der Gasfreisetzungsmechanismus (6) automatisch aktiviert wird.

7. Rückhaltevorrichtung nach einem der obengenannten Ansprüche, wobei der aufblasbare Schlauch (2) durch Freisetzung von Druckgas aus zwei oder mehr Druckgas-Einwegpatronen aufgeblasen wird.

8. Rückhaltevorrichtung nach einem der obengenannten Ansprüche, wobei der aufblasbare Schlauch (2) durch Gas aufgeblasen wird, das von einem Gasgenerator erzeugt wird.

9. Rückhaltevorrichtung nach einem der obengenannten Ansprüche, ferner umfassend eine Mehrzahl von Leuchten, die um einen Außenumfang des Bandes (1) herum positioniert sind.

10. Rückhaltevorrichtung nach einem der obengenannten Ansprüche, ferner umfassend eine Mehrzahl von nicht überlappenden aufblasbaren Schläuchen, die um einen Innenumfang des Bandes (1) herum positioniert sind.

11. Rückhaltevorrichtung nach einem der obengenannten Ansprüche, wobei der aufblasbare Schlauch (2) durch Klettverschlüsse gegenüber der Innenwand des Bandes (1) und um den Umfang des Bandes herum in Position gehalten wird.

12. Rückhaltevorrichtung nach einem der obengenannten Ansprüche, ferner umfassend einen Mechanismus (9) zum Entleeren des aufblasbaren Schlauchs (2).

13. Rückhaltevorrichtung nach einem der obengenannten Ansprüche, ferner umfassend ein Seil (11) oder eine Mehrzahl von Seilen, die um einen Außenumfang des Bandes (1) herum angeordnet sind.

14. Rückhaltevorrichtung nach einem der obengenannten Ansprüche, wobei das Netz (5) das Band (1) hält, wobei der aufblasbare Schlauch (2) in etwa in Taillenhöhe an der Person befestigt ist, wenn das Band (1) über den Kopf, die Arme und den Rumpf der Person geführt wird.

15. Rückhaltevorrichtung nach einem der Ansprüche 1 bis 13, wobei das Netz das Band (1) hält, wobei der aufblasbare Schlauch (2) zwischen in etwa Hüfthöhe und in etwa Brustkorbhöhe an der Person befestigt ist, wenn das Band (1) über den Kopf, die Arme und den Rumpf der Person geführt wird.

16. Rückhaltevorrichtung nach einem der Ansprüche 1 bis 13, wobei eine Tiefe des Netzes (5) zwischen 0,5 m und 1,0 m beträgt.

17. Rückhaltevorrichtung nach einem der obengenannten Ansprüche, wobei das Band, das so bemessen ist, dass es über den Oberkörper und die Schultern einer Person passt, so bemessen ist, dass es einen Innendurchmesser zwischen 0,5 m und 1,0 m aufweist.

## Revendications

1. Dispositif d'immobilisation d'être humain, comprenant :
une bande (1) dimensionnée pour s'ajuster sur le torse supérieur et les épaules d'une personne ;
un tube gonflable (2) fixé à une paroi intérieure de la bande (1), dans lequel le tube se dilate pendant un gonflage vers l'intérieur et loin de la bande (1), et de telle sorte que le tube (2), lorsqu'il est gonflé, appuie contre le torse de la personne ;
un filet (5) comprenant une embouchure fixée autour d'une circonférence de la bande (1) ; et
un mécanisme de libération de gaz (6) pour gonfler rapidement le tube gonflable (2).

2. Dispositif d'immobilisation d'être humain selon la revendication 1, comprenant en outre un masque anti-crachat (10) fixé à une extrémité de poche du filet (5).

3. Dispositif d'immobilisation d'être humain selon la revendication 1 ou la revendication 2, comprenant en outre une corde (11) ou une pluralité de cordes fixées à la bande (1) ou au filet (5), dans lequel, en utilisation, la corde (11) peut être saisie et maintenue par une personne ou des personnes autres que la personne sur laquelle ledit dispositif est ajusté.

4. Dispositif d'immobilisation d'être humain selon l'une quelconque des revendications 1 à 3, dans lequel le mécanisme (6) pour gonfler rapidement le tube gonflable (2) est connecté à une cartouche (7) de gaz comprimé.

5. Dispositif d'immobilisation d'être humain selon la revendication 4, dans lequel le gaz comprimé est du dioxyde de carbone.

6. Dispositif d'immobilisation d'être humain selon l'une quelconque des revendications précédentes, dans lequel le mécanisme de libération de gaz (6) est activé automatiquement.

7. Dispositif d'immobilisation d'être humain selon l'une quelconque des revendications précédentes, dans lequel le tube gonflable (2) est gonflé par libération de gaz comprimé à partir de deux ou plus de deux cartouches jetables de gaz comprimé.

8. Dispositif d'immobilisation d'être humain selon l'une quelconque des revendications précédentes, dans lequel le tube gonflable (2) est gonflé par du gaz produit par un générateur de gaz.

9. Dispositif d'immobilisation d'être humain selon l'une quelconque des revendications précédentes, comprenant en outre une pluralité de lumières positionnées autour d'une circonférence extérieure de la bande (1).

10. Dispositif d'immobilisation d'être humain selon l'une quelconque des revendications précédentes, comprenant en outre une pluralité de tubes gonflables sans chevauchement positionnés autour d'une circonférence intérieure de la bande (1).

11. Dispositif d'immobilisation d'être humain selon l'une quelconque des revendications précédentes, dans lequel le tube gonflable (2) est maintenu en position par rapport à la paroi intérieure de la bande (1) et autour de la circonférence de la bande par des fixations à crochets et à boucles.

12. Dispositif d'immobilisation d'être humain selon l'une quelconque des revendications précédentes, comprenant en outre un mécanisme (9) pour dégonfler le tube gonflable (2).

13. Dispositif d'immobilisation d'être humain selon l'une quelconque des revendications précédentes, comprenant en outre une corde (11) ou une pluralité de cordes positionnée(s) autour d'une circonférence extérieure de la bande (1).

14. Dispositif d'immobilisation d'être humain selon l'une quelconque des revendications précédentes, dans lequel le filet (5) retient la bande (1) avec le tube gonflable (2) fixé à environ la hauteur de la taille sur la personne lorsque la bande (1) est passée au-dessus de la tête, des bras et du torse de la personne.

15. Dispositif d'immobilisation d'être humain selon l'une quelconque des revendications 1 à 13, dans lequel le filet retient la bande (1) avec le tube gonflable (2) fixé entre environ la hauteur de la hanche et la hauteur de la cage thoracique sur la personne lorsque la bande (1) est passée au-dessus de la tête, des bras et du torse de la personne.

16. Dispositif d'immobilisation d'être humain selon l'une quelconque des revendications 1 à 13, dans lequel une profondeur du filet (5) est comprise entre 0,5 mètre et 1,0 mètre.

17. Dispositif d'immobilisation d'être humain selon l'une quelconque des revendications précédentes, dans lequel la bande dimensionnée pour s'ajuster sur le torse supérieur et les épaules d'une personne est dimensionnée pour avoir un diamètre intérieur compris entre 0,5 mètre et 1,0 mètre.
